# EUROPEAN PATENT SPECIFICATION

(11) **EP 3 220 832 B1**
(45) Date of publication and mention of the grant of the patent: **09.01.2019**
(21) Application number: 15781422.9
(22) Date of filing: 03.09.2015
(51) Int. Cl.: A61B 10/00

(54) **CONTAINER FOR COLLECTION AND PRESERVATION OF BIOPSY SAMPLES**
BEHÄLTER ZUR ENTNAHME UND KONSERVIERUNG VON BIOPSIEPROBEN
RÉCIPIENT DE COLLECTE ET DE CONSERVATION D'ÉCHANTILLONS DE BIOPSIE

(30) Priority: 17.11.2014 IT TO20140949
(43) Date of publication of application: 27.09.2017
(73) Proprietor: Traces S.r.l., 10022 Carmagnola (Torino) (IT)
(72) Inventor: TARTARO, Daniele, 10023 Chieri (Torino) (IT); MALETTI, Maurizio, 10040 La Loggia (Torino) (IT)
(74) Representative: Rondano, Davide
(86) International application number: PCT/IB2015/056693
(87) International publication number: WO 2016/079611

(56) References cited:
- EP-A2- 0 573 098
- WO-A1-2012/171529
- WO-A1-2013/141834
- US-A1- 2009 104 692
- US-A1- 2014 051 178

## Description

The present invention relates to a container for collection and preservation of biopsy samples.

As is known, biopsy is a medical examination consisting in taking a portion or fragment of tissue from a living organism to allow analysis of that portion or fragment of tissue (for example by means of a microscope or by means of microbiological techniques or molecular biology techniques) for diagnostic purposes. Once it has been taken from the patient, the tissue sample is collected in a container and then a preserving agent, typically formalin (formaldehyde in water solution), is put into the container to ensure preservation of the biopsy sample contained inside the container. The container, with the biopsy sample immersed in formalin, can then be closed and sent to a laboratory to be analysed. Formalin, as well as other preserving agents, is toxic by inhalation, in particular carcinogenic. Although the operation of introducing formalin into the container is performed under an extractor fan, this operation is however hazardous for the health of the operators assigned to collect the biopsy samples. There is therefore a need to prevent the operator from coming into direct contact with formalin or, in any case, from being exposed to the vapours of the formalin used as preserving agent for preservation of biopsy samples.

In order to fulfil this need suitable containers for collection and preservation of biopsy samples have been conceived. WO2012/171529 discloses a container for collection and preservation of biopsy samples having the features set forth in the preamble of independent claim 1. According to this prior art document, the container comprises a body adapted to contain the biopsy sample to be preserved and a cap adapted to engage the body (for example by screwing onto the body) to close it tightly. The cap comprises a receptacle, which contains a preserving solution (for example formalin) and is sealed off by a membrane, and a puncturing member adapted to be pressed by the operator against the membrane to tear it, thereby allowing the preserving solution contained in the receptacle to flow out of the receptacle and fill the body of the container to submerge the biopsy sample. Such a known container is on the one hand particularly complex, and hence expensive to manufacture, and on the other does not ensure full safety. The operator, in fact, might inadvertently push the puncturing member, while manoeuvring the cap, to such an extent as to tear the membrane and then cause the preserving solution contained in the receptacle to leak out of the latter.

It is therefore an object of the present invention to overcome the above-mentioned drawbacks of the prior art, providing a container for collection and preservation of biopsy samples which ensures higher safety and at the same time is less expensive to manufacture than the prior art.

This and other objects are fully achieved according to the invention by virtue of a container for collection and preservation of biopsy samples having the features defined in the enclosed independent claim 1.

Advantageous embodiments of the invention are the subject-matter of the dependent claims, the contents of which is to be regarded as forming an integral and integrating part of the following description.

In short, the invention is based on the idea of providing a container of the above-identified type, comprising an operating member which is received inside the body of the container and is configured to press the puncturing member against the membrane, until the latter is torn, as a result of the cap being screwed onto the body of the container beyond a predetermined limit. By virtue of such an arrangement, tearing of the membrane, and therefore filling of the body of the container with the preserving solution that flows out of the receptacle, is caused by the operator not in a direct manner by applying pressure onto the puncturing member carried by the cap, as is the case with the prior art discussed above, but in an indirect manner by screwing the cap onto the body of the container. Tearing of the membrane by the puncturing member can thus occur only when the cap is screwed beyond a given extent onto the body of the container, i.e. when the container is already closed and hence there is no possibility for the preserving solution and/or its vapours to leak out of the container. Accordingly, there is no possibility for the operator to inadvertently act on the puncturing member, causing tearing of the membrane, when the cap is disassembled from the container, and hence for the operator to be exposed to the preserving solution due to possible leakages of the preserving solution from the receptacle formed in cap of the container. The container according to the invention ensures therefore higher safety than the prior art against the risk of being exposed to the preserving solution used to preserve the biopsy sample in the container.

Further features and advantages of the present invention will become more apparent from the following detailed description, given purely by way of non-limiting example with reference to the appended drawings, where:
Figures 1 and 2 are perspective views of a container for collection and preservation of biopsy samples according to an embodiment of the present invention, in the initial delivery state and in the final use state, respectively;
Figures 3 and 4 are axial section views of the container in the state of Figure 1 and in the state of Figure 2, respectively;
Figure 5 is an exploded view of the container of Figures 1 and 2;
Figure 6 is a perspective view showing on an enlarged scale the cap of the container of Figures 1 and 2;
Figure 7 is a perspective view showing on an enlarged scale a detail of the cap of Figure 6; and
Figures 8 and 9 are schematic axial section views of a container for collection and preservation of biopsy samples according to a further embodiment of the present invention, in the initial delivery state and in the final use state, respectively.

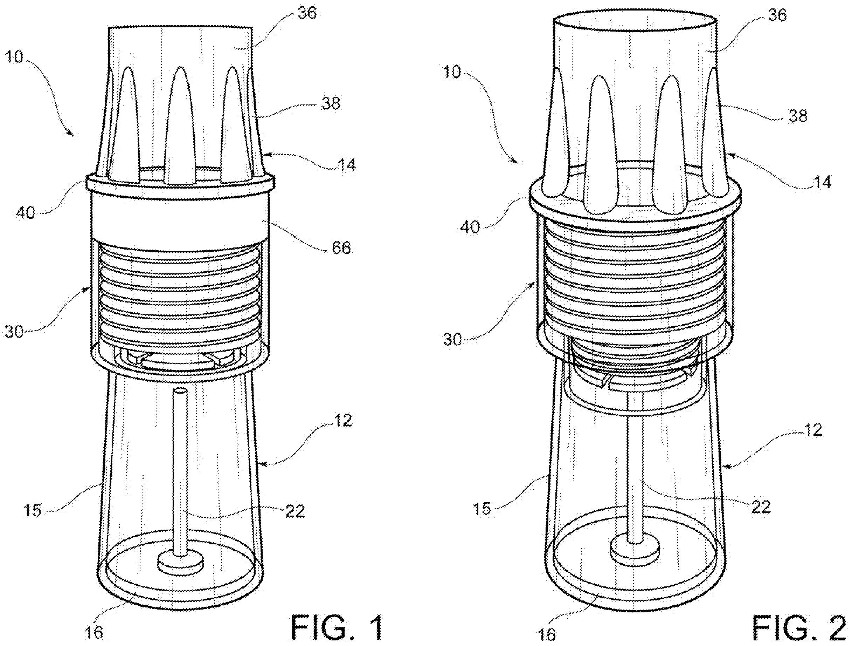

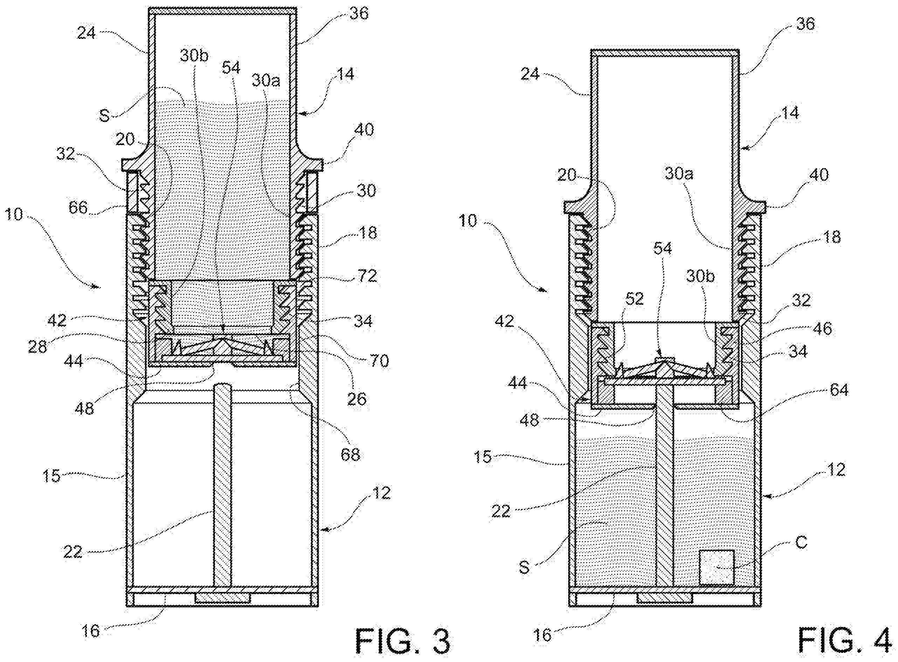

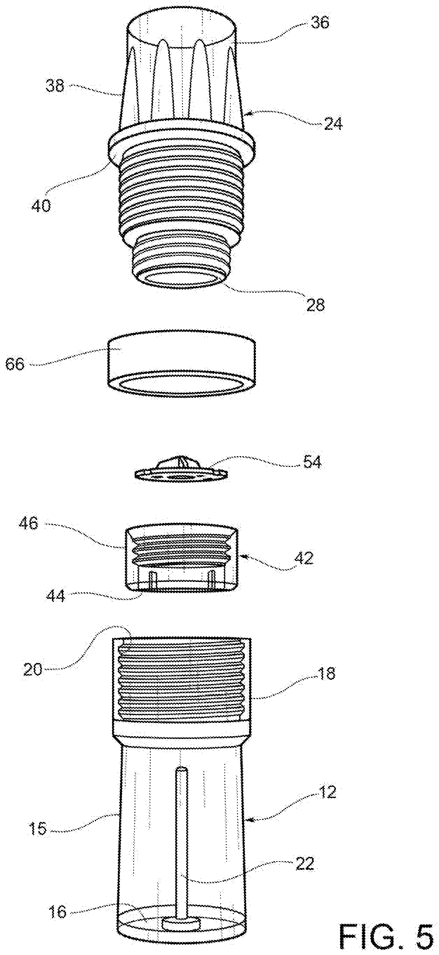

With reference first to Figures 1 to 7, a container for collection and preservation of biopsy samples according to an embodiment of the present invention is generally indicated 10. The container 10 basically comprises a body or cup 12 adapted to contain a biopsy sample to be preserved (schematically shown in Figure 4, where it is indicated C) and a cap 14 adapted to be screwed onto the body 12 to close it tightly.

The body 12 comprises a side wall 15 and is closed at its bottom by a bottom wall 16 on which the biopsy sample C is to be placed. Moreover, the body 12 has at its top a mouth 18 provided with an internal thread 20. An operating member 22 is received inside the body 12, which member is made in the embodiment of Figures 1 to 7 as a rod or column extending coaxially to the body 12 and is attached to the bottom wall 16 of the latter. The body 12 may be pre-filled with an aqueous solution, for example physiological solution, in order to make placing of the biopsy sample inside the body easier.

The cap 14 comprises a receptacle 24 in which a preserving solution S (for example formalin) is contained. The receptacle 24 is suitably dimensioned to contain the desired volume of preserving solution and, preferably, also to leave a free volume equal at least to 30% of the volume of the preserving solution. The receptacle 24 is sealed off at its bottom by a tearable membrane 26, which is made for example of aluminium or polymeric material. The membrane 26 is attached, for example by gluing, to an annular bottom edge 28 of the receptacle 24. The receptacle 24 comprises a lower portion 30 provided with a first external thread 32, adapted to engage with the internal thread 20 of the mouth 18 of the body 12, and with a second external thread 34. The first thread 32 and the second thread 34 are formed in a first section 30a and in a second section 30b of the lower portion 30, respectively, the first section 30a being placed above the second section 30b and having a larger diameter than that of the second section 30b. A lip gasket (not shown), made for example of silicone, may be provided between the first external thread 32 of the cap 14 and the internal thread 20 of the body 12 to ensure tightness to vapours and liquids both in case the body 12 is pre-filled with an aqueous solution and during screwing of the cap 14 onto the body 12 with resulting tearing of the membrane 26 which seals off the receptacle 26. The receptacle 24 further comprises an upper portion 36 which is provided with means 38 (formed for example by radial projections) for increasing grip by the operator and hence making the operation of screwing the cap 14 onto the body 12 easier. An annular flange 40 is provided between the lower portion 30 and the upper portion 36 of the receptacle 24 to act as end-of-travel member when the cap 14 is being screwed onto the body 12.

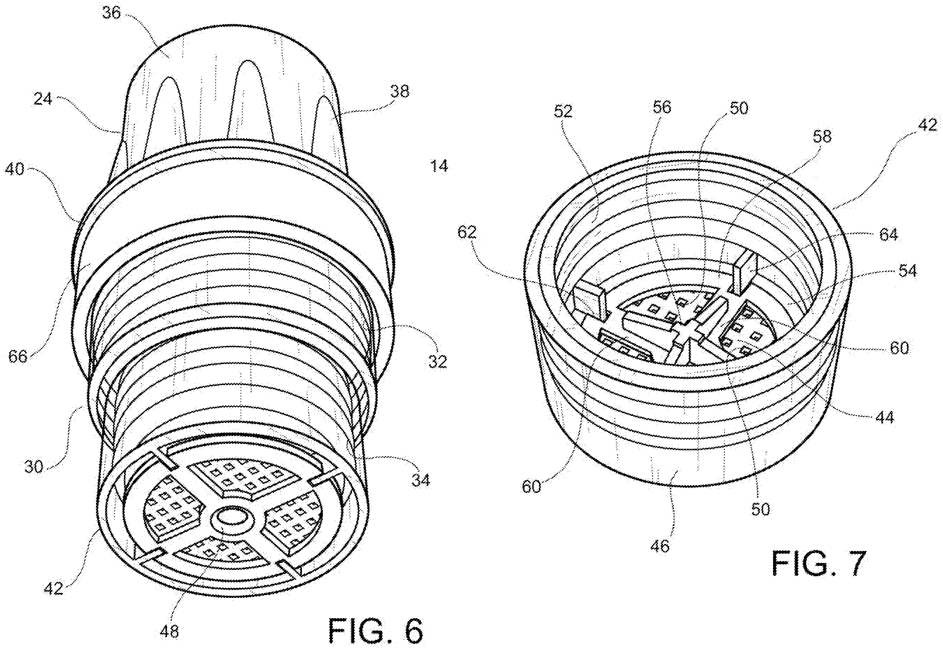

The cap 14 further comprises a separating element, or filter, 42 which is screwed to the lower portion 30 of the receptacle 24. The filter 42 has a glass-like shape, with a circular bottom wall 44 and a cylindrical side wall 46. The bottom wall 44 has a central hole 48, whose diameter is such as to allow the operating member 22 to pass through that hole, as will be explained in detail further on, and a plurality of holes 50 (Figure 7) of smaller diameter, for example between 0,5 and 1,5 mm, to allow the preserving solution to flow from the receptacle 24 to the body 12 when the membrane 26 closing the receptacle 24 is torn. Preferably, no holes are provided in the outermost annular portion of the bottom wall 44, that is to say, the holes 50 are provided only in the area comprised between the central hole 48 and said outermost annular portion, so as to reduce backflow of the preserving solution from the body 12 to the receptacle 24 once the membrane 26 has been torn and the preserving solution has flowed from the receptacle 24 into the body 12. The side wall 46 of the filter 42 is provided with an internal thread 52 adapted to engage with the second external thread 34 of the lower portion 30 of the receptacle 24 to allow the filter 42 to be screwed to the receptacle 24.

A puncturing member 54 is received inside the filter 42 and is adapted to puncture, or tear, the membrane 26 of the receptacle 24 when it is pressed against the latter by the operating member 22. In the illustrated example (see in particular Figure 7) the puncturing member 54 comprises a central punch 56, a ring 58 arranged around the punch 56 and a plurality of connecting arms 60 connecting the punch 56 to the ring 58. The upper end (i.e. the end facing the membrane 26) of the punch 56. has a pointed shape, or at least a shape such as to be able to tear the membrane 26 when the puncturing member 54, and hence the punch 56, is urged against the membrane. The puncturing member 54 is mounted so as to axially slidable (i.e. slidable in the direction of the longitudinal axis of the container 10) inside the filter 42. To this end, the ring 58 of the puncturing member 54 has a plurality of guide slits 62 each engaged by a corresponding guide projection 64 extending radially inwards from the side wall 46 of the filter 42.

In the initial delivery state of the container 10, as shown in Figures 1 and 3, the puncturing member 54 rests against the bottom wall 44 of the filter 42 and the punch 56 is at a certain distance from the membrane 26. Moreover, in the initial delivery state the container 10 is provided with a safety ring 66 arranged around the first thread 32 of the lower portion 30 of the receptacle 24 so as to be axially interposed between the flange 40 of the receptacle 24 and the upper edge of the mouth 18 of the body 12. Due to the presence of the safety ring 66, the cap 14 cannot be screwed further with respect to the initial delivery state, where the flange 40 abuts against the safety ring 66 and the latter abuts in turn against the upper edge of the mouth 18. The container 10 is configured such that in the initial delivery state the puncturing member 54 is positioned at a certain distance from the operating member 22. Since in this state, as explained above, the cap 14 cannot be screwed further onto the body 12, and hence the puncturing member 54 cannot be moved closer to the operating member 22, the puncturing member 54 remains spaced from the membrane 26. There are therefore no risks that the operator, while handling the container 10, may cause tearing of the membrane 26 and hence leakage of the preserving solution out of the container.

On the other hand, once the safety ring 66 has been removed, the cap 14 can be screwed further onto the body 12 with respect to the initial delivery state shown in Figures 1 and 3. In this way, the puncturing member 54 first comes into contact with the upper end of the operating member 22 and then is moved, rising from the bottom wall 44 of the filter 42, against the membrane 26 until it causes tearing of the latter. Figures 2 and 4 show the container 10 in the final use state, in which the operator, after putting the biopsy sample inside body 12 and removing the safety ring 66, has completely screwed the cap 14 onto the body 12 bringing the flange 40 of the receptacle 24 into abutment against the upper edge of the mouth 18 of the body 12.

Once the membrane 26 has been torn, the preserving solution is free to leak, by gravity, out of the receptacle 24, flowing through the holes 50 in the bottom wall 44 of the filter 42 and falling in the underlying body 12.

Advantageously, in order to slow down the flow of the air out of the body 12 when the cap 14 is screwed to the final use state, hence without the safety ring 66, the side wall 15 of the body 12 has a thickened portion 68 defining, along with the side wall 46 of the filter 42, a restriction 70. The restriction 70 allows to reduce the flow section area between the lower portion of the body 12, where the preserving solution with its vapours is present, and the mouth 18. Moreover, the increase in the flow section area in the region of the body 12 above the thickened portion 68 (region indicated 72 in Figure 3) produces, due to the Venturi effect, a pressure drop which also helps to prevent the vapours of the preserving solution from leaking out of the container 10.

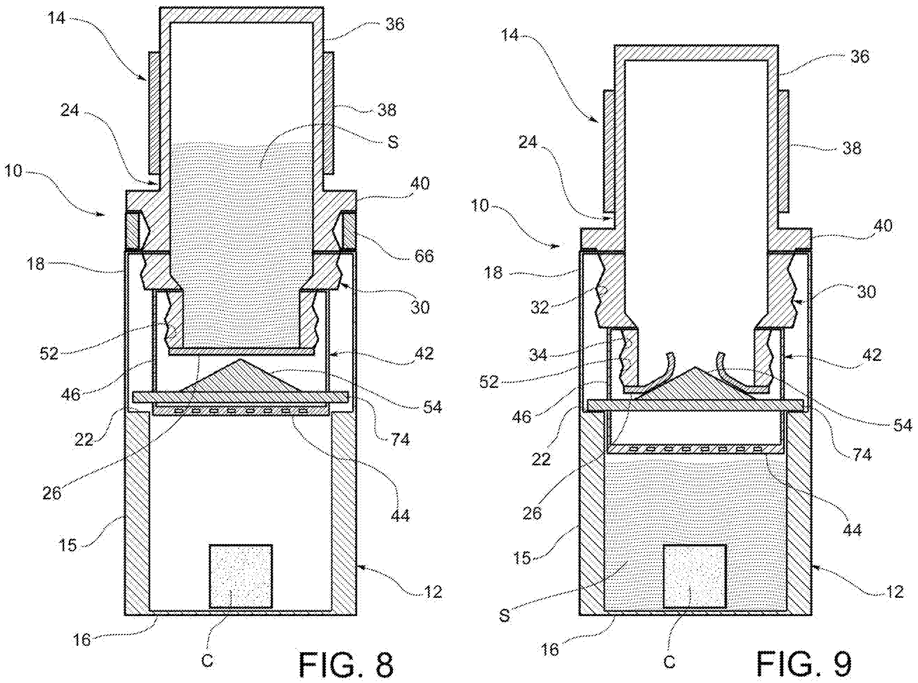

The embodiment described above with reference to Figures 1 to 7 is particularly suitable for biopsy samples of small size. A variant of embodiment of the present invention, conceived to allow collection and preservation also of biopsy samples of larger size, is schematically shown in Figures 8 and 9, where parts and elements identical or corresponding to those of Figures 1 to 7 have been given the same reference numerals.

This further embodiment differs from the previous one in that the operating member 22 is not formed by a rod or column which is placed in the middle of the body 12 and is adapted to pass through a corresponding central hole provided in the bottom wall 44 of the filter 42 to operate the puncturing member 54, but by a shoulder against which a plurality of tabs 74 of the puncturing member 54 radially projecting from the side wall 46 of the filter 42 come into abutment as a result of the cap 14 being completely screwed onto body 12. The shoulder 22 acting as operating member is preferably formed by the side wall 15 of the body 12, as in the illustrated example. For the rest, the structure and operation of the container according to this further embodiment are identical to those described above in connection with the first embodiment.

As is clear from the above description, the present invention allows to collect and preserve biopsy samples avoiding the risk that the operator may come into contact both with the preserving solution and with the vapours and the volatile substances diffused by the preserving solution. Moreover, the risk that the preserving agent may be inadvertently delivered is avoided, or at least significantly reduced with respect to the prior art.

Naturally, the principle of the invention remaining unchanged, the embodiments and the constructional details may vary widely from those described and illustrated purely by way of non-limiting example, without thereby departing from the scope of the invention as defined in the appended claims.

## Claims

1. Container (10) for collection and preservation of biopsy samples (C), comprising a body (12) adapted to contain the biopsy sample (C) and a cap (14) adapted to be screwed onto the body (12) to close it tightly,
wherein the body (12) comprises a side wall (15), a bottom wall (16) and a mouth (18), wherein the cap (14) comprises a receptacle (24) for containing a preserving solution (S), a tearable membrane (26) which seals off the receptacle (24) at its bottom, a puncturing member (54) adapted to be pressed against the membrane (26) to tear it, and a filter (42) which is attached to the receptacle (24) and comprises a bottom wall (44) having a plurality of holes (50) for allowing the preserving solution (S) to flow from the receptacle (24) to the body (12) as a result of tearing of the membrane (26), and
wherein the container (10) further comprises an operating member (22) adapted to control the movement of the puncturing member (54) to press it against the membrane (26), **characterized**
**in that** the receptacle (24) has a first external thread (32) and the mouth (18) has an internal thread (20) adapted to engage said first external thread (32) to allow the cap (14) to be screwed onto the body (12), and
**in that** the operating member (22) is received inside the body (12) and is configured to press the puncturing member (54) against the membrane (26), until the latter is torn, as a result of the cap (14) being screwed onto the body (12) beyond a predetermined limit.

2. Container according to claim 1, further comprising a safety ring (66) releasably arranged around said first external thread (32) to prevent the cap (14) from being screwed onto the body (12) beyond said predetermined limit.

3. Container according to claim 1 or claim 2, wherein the receptacle (24) comprises an annular flange (40) adapted to act as an end-of-travel member when the cap (14) is screwed onto the body (12).

4. Container according to claim 2 and claim 3, wherein the safety ring (66) is axially interposed between the annular flange (40) and an upper edge of the mouth (18).

5. Container according to any of the preceding claims, wherein the receptacle (24) has also a second external thread (34) adapted to engage an internal thread (52) of the filter (42), and wherein said first external thread (32) and said second external thread (34) are formed in a first section (30a) and in a second section (30b) of a lower portion (30) of the receptacle (24), respectively, said first section (30a) being placed above said second section (30b) and having a larger diameter than that of said second section (30b).

6. Container according to any of the preceding claims, wherein the puncturing member (54) is mounted so as to be axially slidable inside the filter (42).

7. Container according to claim 6, wherein the operating member (22) is made as a rod or column which extends coaxially to the body (12) and is attached to the bottom wall (16) of the body (12), and wherein the bottom wall (44) of the filter (42) has a central hole (48) adapted to allow the operating member (22) to pass through said hole (48).

8. Container according to claim 6, wherein the operating member (22) is formed by a shoulder acting as an abutment surface for a plurality of tabs (74) of the puncturing member (54) radially projecting from a side wall (46) of the filter (42).

## Patentansprüche

1. Behälter (10) zur Aufbewahrung und Konservierung von Biopsieproben (C), mit einem Gehäuse (12), das dazu ausgelegt ist, die Biopsieprobe aufzunehmen, und einer Kappe (14), die dazu ausgelegt ist, auf das Gehäuse (12) aufgeschraubt zu werden, um es dicht zu verschließen,
wobei das Gehäuse (12) eine Seitenwand (15), eine Bodenwand (16) und einen Einlass (18) aufweist,
wobei die Kappe (14) ein Gefäß (24) zum Aufnehmen einer Konservierungslösung (S), eine einreißbare Membran (26), die das Gefäß (24) an seinem Boden abdichtet, ein Durchstechelement (54), das geeignet ist, gegen die Membran (26) gedrückt zu werden, um sie einzureißen, und einen Filter (42) aufweist, welcher an dem Gefäß (24) befestigt ist und eine Bodenwand (44) mit einer Vielzahl von Löchern (50) aufweist, welche gestatten, dass die Konservierungslösung (S) infolge des Einreißens der Membran (26) von dem Gefäß (24) zu dem Gehäuse (12) strömt, und
wobei der Behälter (10) weiter ein Betätigungselement (22) aufweist, welches dazu ausgelegt ist, die Bewegung des Durchstechelements (54) zu steuern, um es gegen die Membran (26) zu drücken,
**dadurch gekennzeichnet, dass**
das Gefäß (24) ein erstes Außengewinde (32) aufweist und der Einlass (18) ein Innengewinde (20) aufweist, welches dazu ausgelegt ist, in das erste Außengewinde (32) einzugreifen, damit die Kappe (14) auf das Gehäuse (12) geschraubt werden kann, und
dass das Betätigungselement (22) in dem Gehäuse (12) aufgenommen und dazu ausgelegt ist, das Durchstechelement (54) gegen die Membran (26) zu drücken, bis letztere infolge dessen, dass die Kappe (14) über eine vorbestimmte Grenze hinaus auf das Gehäuse (12) geschraubt wird, einreißt.

2. Behälter nach Anspruch 1, weiter aufweisend einen Sicherheitsring (66), der lösbar um das erste Außengewinde (32) herum angeordnet ist, um zu verhindern, dass die Kappe (14) über eine vorbestimmte Grenze hinaus auf das Gehäuse (12) geschraubt wird.

3. Behälter nach Anspruch 1 oder Anspruch 2, wobei das Gefäß (24) einen Ringflansch (40) aufweist, der dazu ausgelegt ist, als Endelement zu dienen, wenn die Kappe (14) auf das Gehäuse (12) geschraubt wird.

4. Behälter nach Anspruch 2 und Anspruch 3, wobei der Sicherheitsring (66) axial zwischen dem Ringflansch (40) und einer Oberkante des Einlasses (18) angeordnet ist.

5. Behälter nach einem der vorangehenden Ansprüche, wobei das Gefäß (24) ferner ein zweites Außengewinde (34) aufweist, das dazu ausgelegt ist, in ein Innengewinde (52) des Filters (42) einzugreifen, und wobei das erste Außengewinde (32) und das zweite Außengewinde (34) in einem ersten Abschnitt (30a) bzw. in einem zweiten Abschnitt (30b) eines unteren Teils (30) des Gefäßes (24) gebildet sind, wobei der erste Abschnitt (30a) über dem zweiten Abschnitt (30b) angeordnet ist und einen größeren Durchmesser aufweist als der zweite Abschnitt (30b).

6. Behälter nach einem der vorangehenden Ansprüche, wobei das Durchstechelement (54) derart befestigt ist, dass es im Inneren des Filters (42) axial verschiebbar ist.

7. Behälter nach Anspruch 6, wobei das Betätigungselement (22) als Stange oder Säule ausgebildet ist, welche sich koaxial zu dem Gehäuse (12) erstreckt und an der Bodenwand (16) des Gehäuses (12) befestigt ist, und wobei die Bodenwand (44) des Filters (42) eine zentrale Öffnung (48) aufweist, die so angepasst ist, dass das Betätigungselement (22) durch die Öffnung (48) hindurchtreten kann.

8. Behälter nach Anspruch 6, wobei das Betätigungselement (22) durch ein Schulterstück gebildet ist, welches als eine Anlagefläche für eine Vielzahl von Laschen (74) des Durchstosselements (54) dient, welche radial von einer Seitenwand (46) des Filters (42) vorstehen.

## Revendications

1. Contenant (10) de collecte et de conservation d'échantillons de biopsie (C), comprenant un corps (12) adapté pour contenir l'échantillon de biopsie (C) et un capuchon (14) adapté pour être vissé sur le corps (12) pour le fermer hermétiquement,
dans lequel le corps (12) comprend une paroi de côté (15), une paroi de fond (16) et une embouchure (18),
dans lequel le capuchon (14) comprend un réceptacle (24) pour contenir une solution de conservation (S), une membrane déchirable (26) qui colmate le réceptacle (24) au niveau de son fond, un organe de perforation (54) adapté pour être appuyé contre la membrane (26) pour la déchirer, et un filtre (42) qui est fixé au réceptacle (24) et comprend une paroi de fond (44) ayant une pluralité de trous (50) pour permettre à la solution de conservation (S) de s'écouler depuis le réceptacle (24) jusqu'au corps (12) suite au déchirement de la membrane (26), et
dans lequel le contenant (10) comprend en outre un organe de fonctionnement (22) adapté pour commander le mouvement de l'organe de perforation (54) pour l'appuyer contre la membrane (26),
**caractérisé**
**en ce que** le réceptacle (24) comporte un premier filet externe (32) et l'embouchure (18) comporte un filet interne (20) adapté pour s'engager dans ledit premier filet externe (32) pour permettre au capuchon (14) d'être vissé sur le corps (12), et
**en ce que** l'organe de fonctionnement (22) est reçu à l'intérieur du corps (12) et est configuré pour appuyer l'organe de perforation (54) contre la membrane (26), jusqu'à ce que cette dernière soit déchirée, suite au vissage du capuchon (14) sur le corps (12) au-delà d'une limite prédéterminée.

2. Contenant selon la revendication 1, comprenant en outre une bague de sécurité (66) agencée de façon libérable autour dudit premier filet externe (32) pour empêcher le capuchon (14) d'être vissé sur le corps (12) au-delà de ladite limite prédéterminée.

3. Contenant selon la revendication 1 ou la revendication 2, dans lequel le réceptacle (24) comprend une bride annulaire (40) adaptée pour servir d'organe de fin de course lorsque le capuchon (14) est vissé sur le corps (12).

4. Contenant selon la revendication 2 et la revendication 3, dans lequel la bague de sécurité (66) est interposée axialement entre la bride annulaire (40) et un bord supérieur de l'embouchure (18).

5. Contenant selon l'une quelconque des revendications précédentes, dans lequel le réceptacle (24) comporte également un second filet externe (34) adapté pour s'engager dans un filet interne (52) du filtre (42), et dans lequel ledit premier filet externe (32) et ledit second filet externe (34) sont formés dans une première section (30a) et dans une seconde section (30b) d'une portion inférieure (30) du réceptacle (24), respectivement, ladite première section (30a) étant placée au-dessus de ladite seconde section (30b) et ayant un diamètre plus grand que celui de ladite seconde section (30b).

6. Contenant selon l'une quelconque des revendications précédentes, dans lequel l'organe de perforation (54) est monté de façon à être coulissant axialement à l'intérieur du filtre (42).

7. Contenant selon la revendication 6, dans lequel l'organe de fonctionnement (22) est réalisé sous forme de tige ou de colonne qui s'étend coaxialement au corps (12) et est fixé à la paroi de fond (16) du corps (12), et dans lequel la paroi de fond (44) du filtre (42) comporte un trou central (48) adapté pour permettre à l'organe de fonctionnement (22) de passer à travers ledit trou (48).

8. Contenant selon la revendication 6, dans lequel l'organe de fonctionnement (22) est formé par un épaulement servant de surface de butée pour une pluralité de pattes (74) de l'organe de perforation (54) faisant saillie radialement depuis une paroi de côté (46) du filtre (42).
